(19) Europäisches Patentamt European Patent Office Office européen des brevets

(11) **EP 3 490 623 B1**

(12) **FASCICULE DE BREVET EUROPEEN**

(45) Date de publication et mention de la délivrance du brevet:
**17.08.2022 Bulletin 2022/33**

(21) Numéro de dépôt: **17748844.2**

(22) Date de dépôt: **25.07.2017**

(51) Classification Internationale des Brevets (IPC):
**A61L 15/40** (2006.01)

(52) Classification Coopérative des Brevets (CPC):
**A61L 15/40**

(86) Numéro de dépôt international:
**PCT/FR2017/052068**

(87) Numéro de publication internationale:
**WO 2018/020141 (01.02.2018 Gazette 2018/05)**

(54) **DISPOSITIF POUR L'ADSORPTION D'ODEURS**

VORRICHTUNG ZUR ADSORPTION VON GERÜCHEN

DEVICE FOR ADSORBING ODOURS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **26.07.2016 FR 1657156**

(43) Date de publication de la demande:
**05.06.2019 Bulletin 2019/23**

(73) Titulaires:
- **Institut Curie**
  **75248 Paris Cedex 05 (FR)**
- **École Supérieure de Physique et de Chimie Industrielles de la Ville de Paris (ESPCI)**
  **75005 Paris (FR)**
- **Ecole Nationale Superieure de Chimie de Paris**
  **75005 Paris (FR)**
- **Centre National de la Recherche Scientifique (CNRS)**
  **75016 Paris (FR)**
- **Cemag Care**
  **75003 Paris (FR)**

(72) Inventeurs:
- **THULEAU, Aurélie**
  **92300 Levallois Perret (FR)**
- **FROMANTIN, Isabelle**
  **91940 Les Ulis (FR)**
- **SEMETEY, Vincent**
  **72000 Le Mans (FR)**
- **DUGAY, José**
  **91120 Palaiseau (FR)**
- **LEMEUR, Jean-François**
  **35630 Hede (FR)**

(74) Mandataire: **Cabinet Becker et Associés**
  **25, rue Louis le Grand**
  **75002 Paris (FR)**

(56) Documents cités:
**EP-A1- 1 352 927**

- **DATABASE CA [Online] CHEMICAL ABSTRACTS SERVICE, COLUMBUS, OHIO, US; 30 juin 2016 (2016-06-30), J H Kim: "Manufacturing method of deodorant using turmeric", XP002768326, Database accession no. 165:61046 & KR 1 622 250 B1 18 mai 2016 (2016-05-18)**
- **DATABASE CA CHEMICAL ABSTRACTS SERVICE, COLUMBUS, OHIO, US; 12 novembre 2015 (2015-11-12), G Cheng: "A cat litter added with kelp with modified stalks as basic level and the producing method thereof", XP002768325, Database accession no. 163:606120 & CN 104 521 779 A 22 avril 2015 (2015-04-22)**

EP 3 490 623 B1

## Description

L'invention a trait à un dispositif pour diminuer les odeurs, et notamment les odeurs corporelles, comprenant au moins une épice adsorbante. L'invention a plus particulièrement pour objet un dispositif à usage médical ou paramédical pour l'adsorption d'odeurs corporelles, telles que celles émanant de nécrose, de pus, de défécations, d'urine, d'exsudats, de sang, de la sueur, etc. L'invention a également pour objet l'utilisation d'un tel dispositif sous forme de pansement, couche, protection hygiénique, compresse absorbante ou de protège-slip pour adsorber et masquer les odeurs.

De nombreuses odeurs sont susceptibles d'émaner en permanence d'un corps humain ou animal. Parmi ces odeurs, certaines peuvent être perçues de manières négatives par l'individu dont elles émanent et/ou son entourage. La sensation désagréable, voire répulsive, associée à de telles odeurs peut avoir un impact sociologique dramatique dans la vie d'un individu. C'est le cas notamment des mauvaises odeurs émanant de plaies chroniques, dont la prégnance entraine un inconfort considérable pour les patients comme pour leur entourage. Dans une moindre mesure, les personnes souffrant d'incontinence urinaire et/ou fécale, ou ayant subi une stomie intestinale ou digestive peuvent également connaitre un inconfort lié à l'émanation de mauvaises odeurs.

Différents dispositifs ont été développés, principalement à base de charbon actif, pour permettre d'adsorber les odeurs et d'améliorer la qualité de vie des individus confrontés à de tels problèmes d'émanation non contrôlable d'odeurs et dont la cause ne peut pas forcément être traitée rapidement. On connait notamment un pansement, destiné à être appliqué sur une plaie malodorante, composé de charbon actif recouvert d'une enveloppe en nylon non tissé (Actisorb®). Le charbon actif, bien que connu pour ses propriétés adsorbantes, ne donne cependant pas toujours satisfaction dans le cas d'odeurs très marquées, telles que les odeurs émanant de plaies nécrotiques, infectées ou tumorales.

Le document EP 1 352 927 divulgue un dispositif destiné à éliminer les odeurs corporelles comprenant une couche absorbante comprenant une épice en poudre comme par exemple la cannelle, le clou de girofle, ou encore le curcuma et une couche perméable aux liquides. Dans ce dispositif, le liquide est absorbé par le matériau qui contient la poudre végétale.

Face à l'incapacité des dispositifs actuels à contrôler de manière satisfaisante l'émanation d'odeurs nauséabondes, notamment dans certaines situations cliniques, il existe donc un réel besoin d'une solution alternative (et non antibiotique) permettant d'améliorer le confort des personnes susceptibles d'émettre de telles odeurs et de leur entourage.

## Résumé de l'invention

En travaillant sur cette problématique, les inventeurs ont découvert de manière surprenante que certaines épices présentent des capacités adsorbantes particulièrement intéressantes qui permettent de diminuer de manière très satisfaisante les mauvaises odeurs et notamment les odeurs émanant de plaies nauséabondes. Par ailleurs, contrairement au charbon actif, les épices permettent non seulement d'adsorber une grande partie des émanations odorantes, mais également de couvrir ou masquer celles qui pourraient ne pas être entièrement adsorbées. Les inventeurs ont ainsi mis au point un dispositif intégrant au moins une épice, sous forme de poudre, adsorbant les odeurs et permettant, lorsqu'il est au contact ou à proximité de la source de mauvaises odeurs, de réduire voire supprimer l'inconfort de l'utilisateur et de son entourage vis-à-vis de ces odeurs. C'est notamment le cas d'épices telles que la cannelle, le curcuma ou le clou de girofle qui, sous forme de poudre, présentent des propriétés adsorbantes et masquantes particulièrement adaptées à supprimer ou réduire fortement les odeurs corporelles nauséabondes et la gêne associée à leurs émanations.

L'invention a donc pour objet un dispositif pour l'adsorption d'odeurs comprenant une couche absorbante et une couche adsorbante, ladite couche adsorbante étant munie d'un matériau perméable aux composés organiques volatils (COVs) et d'une charge particulaire adsorbante comprenant au moins une épice, choisie parmi la cannelle, le curcuma et le clou de girofle, ledit matériau perméable aux COVs étant un textile tissé ou non tissé, ledit dispositif comprenant entre 0,5 et 5 g d'épices pour 100 cm2 de matériau perméable aux COVs, ladite épice étant maintenue sous forme de poudre dans ledit dispositif.

## Description des figures

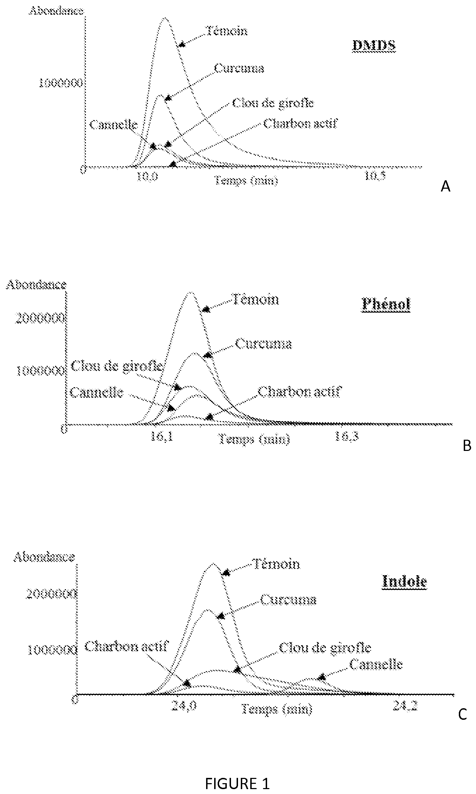

Figure 1 : Comparaison des propriétés adsorbantes du charbon actif (Actisorb®), d'une poudre de cannelle, d'une poudre de curcuma et d'une poudre de clou de girofle, susceptibles d'être utilisés dans le dispositif selon l'invention, sur trois COVs caractéristiques des nécroses malodorantes, le diméthyldisulfure - DMDS (A), le phénol - Ph (B) et l'indole - In (C), le Témoin représentant le COV considéré (100% d'abondance) ;

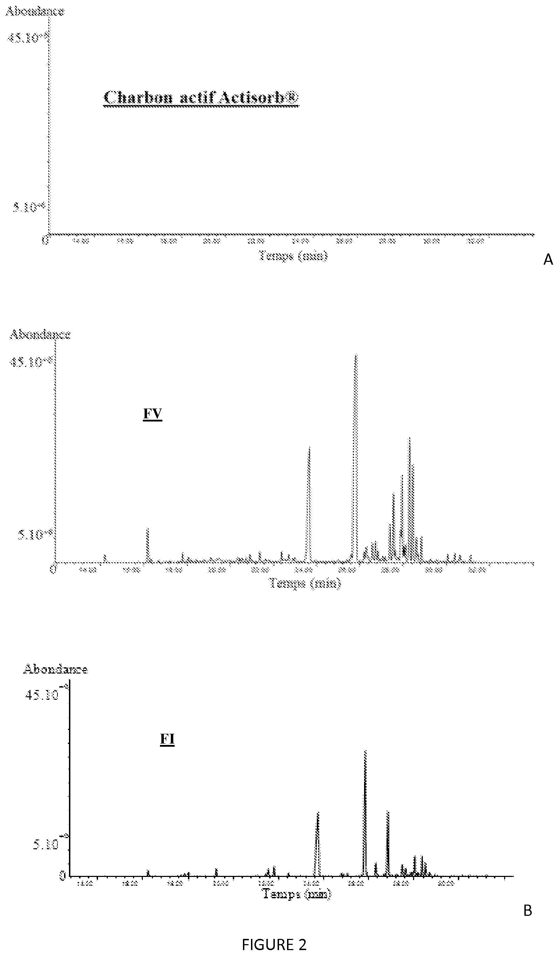

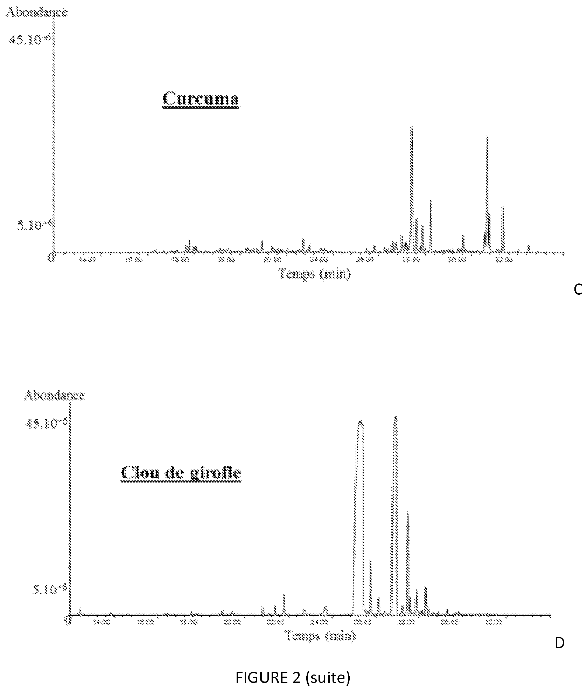

Figure 2 : Comparaison des COVs émis par (A) du charbon actif Actisorb®, (B) deux poudres de cannelle de différentes provenances FV (cannelle de Ceylan) et FI, (C) une poudre de curcuma et (D) une poudre de clou de girofle, susceptibles d'être utilisés dans le dispositif selon l'invention. Les profils chromatographiques ont été obtenus par une méthode d'analyse couplant une microextraction sur phase solide (SPME) en mode espace de tête (HS) gérée par un automate (bras passeur d'échantillons), à une chromatographie en phase gazeuse (GC) avec détection par spectrométrie de masse simple quadripole (MS) ;

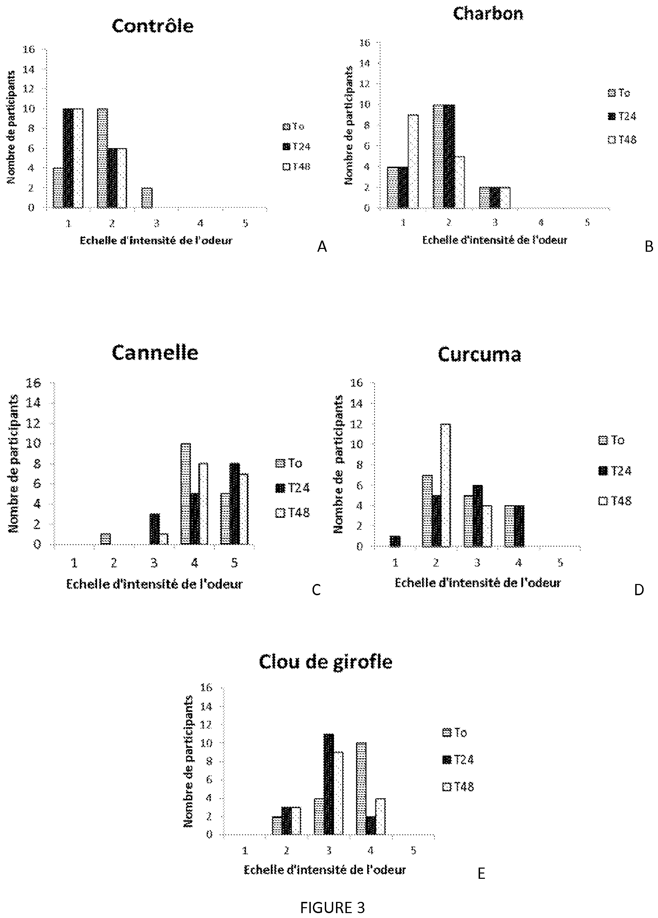

Figure 3 : Tests sensoriels à l'aveugle d'évaluation de l'intensité de l'odeur sur un panel d'utilisateurs associée à des COVs malodorants : (A) Contrôle ; (B) charbon actif (Actisorb®) ; (C) poudre de cannelle (Cannelle de Ceylan) ; (D) poudre de Curcuma *(Curcuma longa)* ; (E) poudre de clou de girofle à T0, T24 heures et T48 heures après application sur du fromage Maroille ;

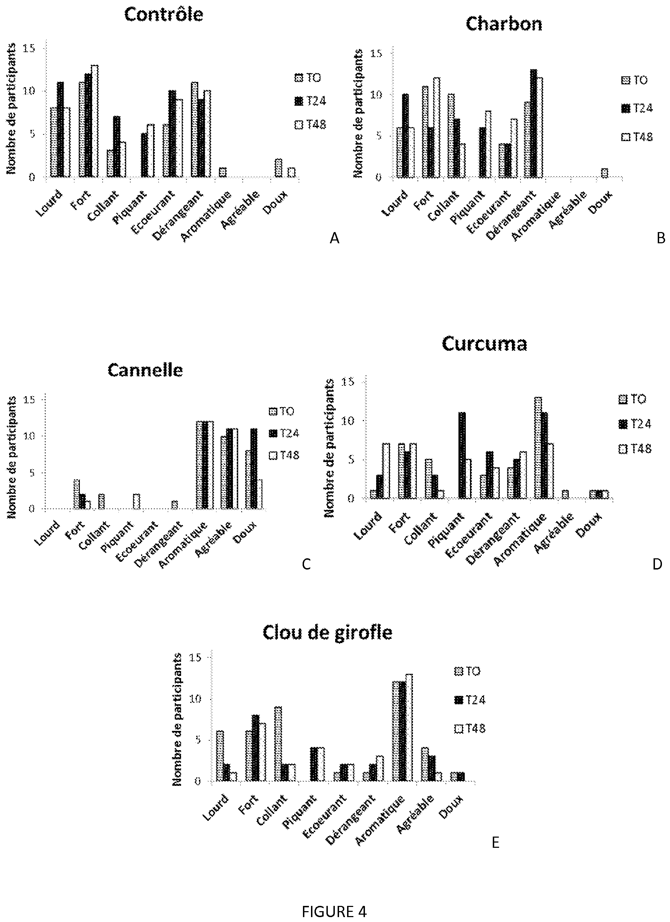

Figure 4 : Tests sensoriels à l'aveugle de qualification de l'odeur associée à des COVs malodorants sur un panel d'utilisateurs : (A) Contrôle ; (B) charbon actif (Actisorb®) ; (C) poudre de cannelle (Cannelle de Ceylan) ; (D) poudre de Curcuma *(Curcuma longa)* ; (E) poudre de clou de girofle à T0, T24 heures et T48 heures après application sur du fromage Maroille ;

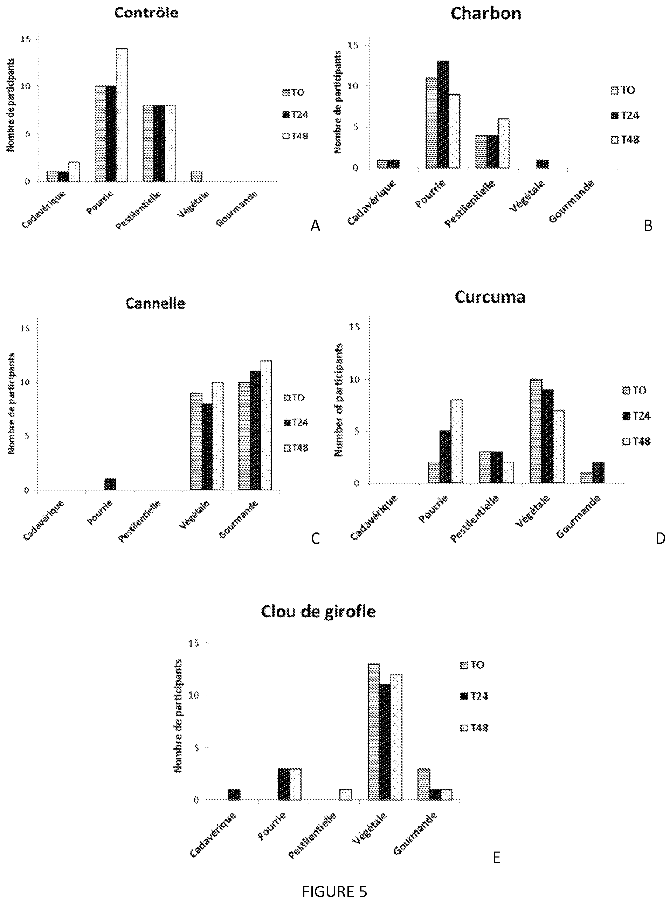

Figure 5 : Tests sensoriels à l'aveugle d'association de l'odeur associée à des COVs malodorants sur un panel d'utilisateurs : (A) Contrôle ; (B) charbon actif (Actisorb®) ; (C) poudre de cannelle (Cannelle de Ceylan) ; (D) poudre de Curcuma *(Curcuma longa)* ; (E) poudre de clou de girofle à T0, T24 heures et T48 heures après application sur du fromage Maroille.

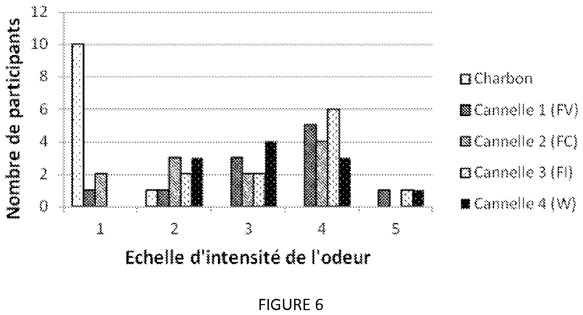

Figure 6 : Tests sensoriels à l'aveugle d'évaluation de l'intensité de l'odeur sur un panel d'utilisateurs associée à des COVs malodorants à T0, T24 heures et T48 heures après application sur du fromage Maroille de charbon actif (Actisorb®), ou différentes poudres de cannelle (FV, FC, FI et W).

**Description détaillée**

L'invention est principalement basée sur la découverte que des épices peuvent remplacer de manière avantageuse le charbon actif, aujourd'hui très largement utilisé comme agent adsorbant, dans un dispositif pour l'adsorption d'odeurs, afin d'adsorber et masquer les odeurs et tout particulièrement les odeurs corporelles. Plus particulièrement, l'invention propose d'utiliser au moins une épice sous forme de poudre, et préférentiellement la cannelle, le clou de girofle et/ou le curcuma, comme agent adsorbant pour réduire les odeurs, notamment en intégrant la poudre d'épice(s) dans un dispositif destiné à être mis au contact ou à proximité d'une source d'odeurs nauséabondes. Selon l'invention, l'épice est intégrée et maintenue dans le dispositif sous forme de poudre.

Dans le contexte de l'invention, une « épice » désigne une substance odorante d'origine végétale.

Le principe d'adsorption est un phénomène de surface par lequel des atomes ou des molécules de gaz ou de liquide se fixent sur une surface solide, adsorbante. Dans le contexte de l'invention, on désigne par « adsorbant » la capacité à fixer des composés organiques volatiles (COVs), de sorte que ces COVs ne se propagent pas ou que faiblement dans l'air. Les odeurs associées à l'émanation de ces COVs sont alors réduites, voire supprimées. Plus particulièrement, une « épice adsorbante » s'entend d'une épice qui, sous forme de poudre, est apte à adsorber au moins 20%, 30%, 40%, préférentiellement au moins 50%, plus préférentiellement au moins 60% d'au moins un COV parmi le diméthyldisulfure (DMDS), phénol (Ph) et indole (In). Dans le contexte de l'invention, le pourcentage, ou taux d'adsorption d'une épice est mesuré en déposant ladite épice dans un tube contenant les COVs et par référence à un tube témoin contenant également les 3 COVs mais ne contenant aucune épice, selon la formule :

$$\text{taux d'adsorption} = [(\text{aire du pic COV tube témoin}) - (\text{aire du pic COV tube avec épice})] / (\text{aire du pic COV tube témoin})$$

Selon l'invention, les profils chromatographiques sont obtenus par une méthode chromatographique couplant une micro-extraction sur phase solide (SPME) en mode espace tête (HS) à une chromatographie en phase gazeuse (GC) associée à une détection par spéctrométrie de masse (MS) (couplage HS-SPME-GC-MS) (Preti et al., Journal of Chromatography B 2009 ; vol. 877, pp. 2011-2018 ; Rey et al., International Journal of Greenhouse Gas Control 2013, vol. 19, no. 11, pp. 576-583).

Par « charge particulaire », ou « poudre », on entend une matière solide, sous forme de particules macroscopiques. Selon l'invention, la charge particulaire comprend ou est constituée d'une épice, sous forme de poudre dont la granulométrie est avantageusement inférieure à 500$\mu$m.

La « granulométrie » s'entend de la distribution des tailles des particules de la charge particulaire, et plus particulièrement de l'épice, considérée. La granulométrie peut notamment se mesurer par tamisage à sec, tamisage humide, sédimentométrie, diffraction laser, microscopie, etc. La granulométrie moyenne correspond au diamètre moyen des particules calculé à partir d'une distribution en volume. Plus précisément, le diamètre moyen arithmétique $(d_a)$ a été calculé, pour un échantillon fractionné en n classes de diamètres représentatifs $d_i$, selon la formule :

$$d_a = D(4,3) = \frac{\sum_{i=1}^{n} d_i{}^4 N_i}{\sum_{i=1}^{n} d_i{}^3 N_i}$$

i = classe des particules, $N_i$ = nombre ou pourcentage des particules dans la classe

Dans un mode de réalisation, la charge particulaire est constituée d'une poudre d'épice dont la granulométrie moyenne est inférieure à 1000$\mu$m, 900$\mu$m, 800$\mu$m, 700$\mu$m, 600$\mu$m, 500$\mu$m, 400$\mu$m, 300$\mu$m, 200$\mu$m, 150$\mu$m, 100$\mu$m, 90$\mu$m, 80$\mu$m, 70 $\mu$m, 60 $\mu$m, 50 $\mu$m, 40 $\mu$m. Notamment, la charge particulaire peut être comprise entre 10$\mu$m et 1000$\mu$m, 700 et 900$\mu$m, 800 et 900$\mu$m, 40 et 400 $\mu$m, entre 50 et 380 $\mu$m, entre 40 et 100 $\mu$m, entre 50 et 60 $\mu$m. Par exemple, la charge particulaire a une granulométrie moyenne de 52 $\mu$m, +/- 10%. Dans un mode de réalisation, la granulométrie moyenne de la charge particulaire n'est pas inférieure à 70$\mu$m, 80$\mu$m, 90 $\mu$m, 100$\mu$m, 150$\mu$m. Dans un exemple, la charge particulaire a une granulométrie comprise entre 60 et 100$\mu$m. Dans un autre exemple, la charge particulaire a une granulométrie moyenne de 90$\mu$m, +/- 10%. Dans un autre mode de réalisation, la granulométrie moyenne de la charge particulaire n'est pas inférieure à 700$\mu$m, 750$\mu$m, 800$\mu$m, 850$\mu$m, 900$\mu$m. Dans un exemple, la charge particulaire a une granulométrie comprise entre 800 et 900$\mu$m, préférentiellement entre 800 et 850$\mu$m. Dans un autre exemple, la charge particulaire a une granulométrie moyenne de 850$\mu$m, +/- 10%.

Selon l'invention, la granulométrie donnée correspond à la granulométrie obtenue avec l'analyseur laser granulométrique Beckman Coulter LS 13320 (Beckman Coulter, USA) en utilisant de l'air comme solvant.

Les inventeurs ont mis en évidence que les épices, et plus particulièrement les épices sous forme de poudre, ont la capacité d'adsorber un grand nombre de COVs potentiellement malodorants afin de diminuer l'impression nauséabonde associée à leur présence. De manière intéressante, ces épices peuvent également permettre de masquer les COVs par la présence de leurs propres COVs, dont l'odeur peut prendre le pas sur les autres.

La charge particulaire de la couche adsorbante du dispositif selon l'invention comprend de la poudre de cannelle, de clou de girofle et/ou du curcuma, plus préférentiellement de cannelle.

Dans un mode de réalisation particulier, la charge particulaire de la couche adsorbante du dispositif selon l'invention comprend de la poudre de cannelle ou de la poudre de curcuma ayant une granulométrie comprise entre 40 et 100 $\mu$m, 60 et 100$\mu$m, ou 80 et 90$\mu$m, +/- 10%. Avantageusement, la charge particulaire de la couche adsorbante du dispositif selon l'invention comprend de la poudre de cannelle ayant une granulométrie comprise entre 50 et 60 $\mu$m. Notamment, la charge particulaire de la poudre de cannelle a une granulométrie moyenne de 52 $\mu$m, +/- 10%.

Dans un mode de réalisation particulier, la charge particulaire de la couche adsorbante du dispositif selon l'invention comprend de la poudre de clou de girofle ayant une granulométrie comprise entre 10 et 1000$\mu$m, Dans un mode de réalisation particulier, la poudre de clou de girofle a une granulométrie comprise entre 800 et 900$\mu$m, préférentiellement entre 800 et 850$\mu$m, +/- 10%.

Le dispositif comprend entre 0.5 et 5g d'épices pour 100 cm$^2$ de matériau perméable aux COVs, préférentiellement entre 0,3 et 5g, encore plus préférentiellement entre 0,5 et 2g, entre 0,5 et 1,5g, entre 0,5 et 1g. Par exemple, le dispositif comprend entre 0,5 et 1,5g de cannelle pour 100 cm$^2$ de matériau perméable aux COVs, et notamment environ 0,9 g de cannelle pour 100 cm$^2$ de matériau perméable aux COVs. Dans un mode de réalisation, le dispositif comprend entre 0,5 et 1g d'épices pour 100 cm$^2$ de matériau perméable aux COVs. Dans un mode de réalisation, le dispositif comprend entre 1 et 4 g, préférentiellement entre 2 et 3 g, plus préférentiellement 2g d'épices, et notamment de cannelle, pour 100 cm$^2$ de matériau perméable aux COVs.

Le matériau perméable aux COVs de la couche adsorbante peut être tout textile tissé ou non tissé. Par exemple, le matériau perméable aux COVs de la couche adsorbante est du nylon (poly(hexaméthylène adipamide)), du coton, du lin, de la viscose, du polyéthylène, du polypropylène et/ou du polyester. Avantageusement, la charge particulaire est imbriquée dans et/ou entre les fibres du textile tissé ou non tissé.

Dans un mode de réalisation particulier, le matériau perméable aux COVs de la couche adsorbante est un textile présentant des formes géométriques, tel qu'un quadrillage, des alvéoles, un matelasse, obtenues notamment par surpiquage, soudure, collage, etc. du textile ou du fait même de la trame dudit textile. Avantageusement, les formes sont réparties de manière régulière sur toute la surface du textile et forment des compartiments dans le volume desquels la charge particulaire est retenue indépendamment du site d'utilisation ou de la gravité. Dans un mode de réalisation particulier, le textile est un textile alvéolé (tel qu'un textile en nid d'abeille). Dans un autre exemple de réalisation, le textile est un textile quadrillé. Ainsi, quelle que soit la position de la couche adsorbante par rapport à l'utilisateur, la charge particulaire est maintenue dans les compartiments et reste répartie de manière homogène sur toute la surface du textile. Plus particulièrement, l'utilisation d'un tel textile compartimenté permet d'éviter que des amas de poudre se forment de manière hétérogène, en particulier dans les coins ou dans une partie du dispositif lors de la manipulation et/ou en cours d'utilisation, du fait notamment de la gravité. Le dispositif selon l'invention est particulièrement adapté pour l'adsorption d'odeurs corporelles, et notamment dans le cadre d'utilisations médicales et paramédicales, pour adsorber les odeurs pouvant émaner de liquides corporels, tels que des exsudats, urine, défécations etc. Le dispositif selon l'invention est tout particulièrement adapté pour l'adsorption d'odeurs émanant de plaies chroniques, et notamment de plaies tumorales d'où émanent un très grand nombre de COVs malodorants, tels que des composés organo-sulfurés (tels que le diméthyldisulfure DMDS, diméthyltrisulfure DMTS), des phénols et des indoles.

Selon l'invention, le dispositif comporte une couche absorbante, destinée à absorber le liquide ou l'humidité susceptible d'être présent dans l'environnement d'où émanent les mauvaises odeurs. Ainsi, seuls les COVs traversent en tout ou partie cette première couche et vont pouvoir être captés par la seconde couche adsorbante, dont les propriétés adsorbantes ne sont ainsi pas perturbées par la présence de liquide.

Tout matériau absorbant non occlusif peut être utilisé pour cette première couche. Dans un mode de réalisation, la couche absorbante comprend au moins un matériau perméable ou semi-perméable, préférentiellement choisi parmi les alginates, la viscose, le carboxyméthylcellulose (CMC), le coton hydrophile ou cellulose, les hydrogels tels que le polyacrylate de sodium, le polyvinyle de pyrrolidine, le collagène, l'acide hyaluronique, le chitosane, l'alginate, gélatine, et des éponges ou mousse tels que la mousse de polyuréthane ou composites de ces matériaux.

Le dispositif selon l'invention peut par exemple être un pansement, une couche, une protection hygiénique, une compresse ou un protège-slip. La couche absorbante est alors destinée à être en contact avec la source de fluide corporel pour absorber au moins partiellement l'humidité. Le dispositif selon l'invention peut également être un pansement de type « pansement secondaire », destiné à être apposé sur un pansement lui-même en contact direct avec la source de fluide corporel. Dans un tel mode de réalisation, le dispositif selon l'invention n'entre pas en contact direct avec la source de fluide corporel.

Dans un mode de réalisation, le dispositif comprend en outre une couche protectrice, préférentiellement semi-perméable ou imperméable à l'humidité, disposée de manière à ce que celle-ci soit prise en sandwich entre la couche absorbante et la couche adsorbante. La couche protectrice permet en particulier d'empêcher que les exsudats remontent à la couche adsorbante, et inversement que les épices diffusent vers la couche absorbante, et que la couche adsorbante soit contaminée par des liquides extérieurs. Une telle couche protectrice peut notamment être un textile tissé ou non tissé, un film ou une membrane microporeuse. Dans un mode de réalisation particulier, la couche protectrice est composée d'un matériau préférentiellement choisi parmi le polyéthylène, le polypropylène, le polyuréthane, le polyester, le polyamide, le polydiméthylsiloxane, les fluoropolymères et/ou composites de ces matériaux.

L'invention a pour objet un pansement comprenant une charge particulaire à base de cannelle. Le pansement comprend une première couche absorbante destinée par exemple à être appliquée sur une plaie chronique, telle qu'une plaie tumorale, et une seconde couche adsorbante constituée d'un textile tissé ou non tissé comprenant de la poudre de cannelle, de granulométrie préférentiellement comprise entre 80 et 90 $\mu$m, +/-10%. Préférentiellement, un tel dispositif comprend entre 0,5 et 2g, préférentiellement entre 1 et 1,5g, de cannelle pour 100 cm$^2$ de matériau perméable aux COVs.

L'invention a également pour objet un pansement comprenant une première couche absorbante destinée par exemple à être appliquée sur une plaie chronique, telle qu'une plaie tumorale, et une seconde couche adsorbante constituée d'un textile tissé ou non tissé comprenant de la poudre de cannelle, de granulométrie comprise entre 50 et 60 $\mu$m, notamment égale à 52 $\mu$m +/-10%, ledit dispositif comprenant entre 1 et 4g, et notamment environ 2 g de cannelle pour 100 cm$^2$ de matériau perméable aux COVs.

L'invention a également pour objet un pansement comprenant une charge particulaire à base de clou de girofle. Le pansement comprend une première couche absorbante destinée par exemple à être appliquée sur une plaie chronique, telle qu'une plaie tumorale, et une seconde couche adsorbante constituée d'un textile tissé ou non tissé comprenant de la poudre de clou de girofle, de granulométrie préférentiellement comprise entre 10 et 1000 $\mu$m, et notamment entre 800 et 900 $\mu$m, plus préférentiellement entre 800 et 850 $\mu$m +/- 10%, par exemple environ 820 $\mu$m. Préférentiellement, un tel dispositif comprend entre 0,5 et 2g, ou entre 1 et 4g, préférentiellement entre 1 et 1,5g, ou entre 2 et 3 g de clou de girofle pour 100 cm$^2$ de matériau perméable aux COVs.

La couche adsorbante comprend entre 0,5 et 5 g, préférentiellement entre 1 et 4 g, plus préférentiellement 2g d'épices pour 100 cm$^2$ de matériau perméable aux COVs.

L'invention a également pour objet un pansement comprenant une charge particulaire à base de curcuma, et notamment à base de *Curcuma longa.* Le pansement comprend une première couche absorbante destinée par exemple à être appliquée sur une plaie chronique, telle qu'une plaie tumorale, et une seconde couche adsorbante constituée d'un textile tissé ou non tissé comprenant de la poudre de curcuma, de granulométrie préférentiellement comprise entre 50 et 150 $\mu$m, préférentiellement entre 80 et 100 $\mu$m, plus préférentiellement égale à 90 $\mu$m +/- 10%. Préférentiellement, un tel dispositif comprend entre 0,5 et 2g, préférentiellement entre 1 et 1,5g, de curcuma pour 100 cm$^2$ de matériau perméable aux COVs.

L'invention a également pour objet un pansement comprenant une charge particulaire à base de curcuma tel que décrit ci-dessus, dans lequel la granulométrie de la poudre de curcuma est comprise entre 50 et 150 $\mu$m, préférentiellement entre 80 et 100$\mu$m, plus préférentiellement égale à 90$\mu$m +/- 10%, ledit dispositif comprenant entre 1 et 4g, et notamment environ 2 g de curcuma pour 100 cm$^2$ de matériau perméable aux COVs.

Les inventeurs ont en effet mis en évidence que la cannelle, le cou de girofle et le curcuma sont particulièrement efficaces dans le cas de plaies tumorales, réputées comme particulièrement malodorantes. Plus précisément, ces épices, sous forme de poudre, présentent des propriétés adsorbantes comparables à celles du charbon actif (Actisorb®) sur au moins trois COVs caractéristiques des plaies tumorales, à savoir le DMDS, le phénol et l'indole (figures 1A, 1B, 1C). En outre, ces épices présentent des propriétés olfactives, et notamment des COVs, propices à masquer les COVs qui pourraient persister et être source d'émanations odorantes nauséabondes. De tels COVs sont absents dans les dispositifs à base de charbon actif (figures 2A, 2B, 2C, 2D) rendant ces épices plus performantes pour résoudre les problèmes liés aux mauvaises odeurs.

Selon l'invention, la poudre d'épice utilisée est préférentiellement apte à adsorber au moins 20%, 30%, 40%, préférentiellement au moins 50%, plus préférentiellement au moins 60% d'au moins un des trois COVs parmi le DMDS, le phénol et l'indole, et au moins 20%, 30%, préférentiellement au moins 40% des deux autres COVs.

De manière alternative, la poudre d'épice est apte à adsorber au moins 20%, 30%, 40%, préférentiellement au moins 50% des trois COVs, DMDS, le phénol et l'indole.

Dans un mode de réalisation, la poudre d'épice utilisée est de la poudre de cannelle apte à adsorber au moins 20%, 30%, 40%, préférentiellement au moins 50% d'au moins un des trois COVs parmi le DMDS, le phénol et l'indole, et au moins 20%, 30%, préférentiellement au moins 40% des deux autres COVs.

Dans un autre mode de réalisation, la poudre d'épice utilisée est de la poudre de cannelle apte à adsorber au moins 65% d'au moins un des trois COVs parmi le DMDS, le phénol et l'indole, et au moins 75% des deux autres COVs.

Dans un autre mode de réalisation, la poudre d'épice utilisée est de la poudre de cannelle apte à adsorber au moins 20%, 30%, 40%, préférentiellement au moins 50% des trois COVs : DMDS, le phénol et l'indole.

Dans un mode de réalisation, la charge particulaire dans le dispositif selon l'invention comprend ou consiste en de la *cannelle de Ceylan (Cinnamomum verum ou Cinnamomum zeylanicum),* de la famille des Lauraceae. Une telle cannelle est disponible dans le commerce. Il est également possible d'utiliser de la cannelle de Chine *(Cinnamomum aromaticum),* de la cannelle du Vietnam ou de Saigon (dite également de Cochinchine) *(Cinnamomum loureiroi),* de la cannelle d'Indonésie *(Cinnamomum burmannii)* ou de la cannelle d'Inde *(Cinnamomum tamala).*

Dans un mode de réalisation particulier, la charge particulaire dans le dispositif selon l'invention comprend ou consiste en de la poudre de cannelle libérant au moins le COV (E)-cinnamaldéhyde. Dans un autre mode de réalisation particulier, la poudre de cannelle utilisée comme charge particulaire libère au moins le COV (E)-Cinnamaldéhyde et au moins un COV parmi a-copaene, $\gamma$-muurolene, $\alpha$-muurolene, a-selinene, $\delta$-cadinene, sativene, $\alpha$-bergamotene, D-germacrene, préférentiellement au moins un COV parmi a-copaene, $\gamma$-muurolene, a-muurolene et $\delta$-cadinene. Dans un mode de réalisation préféré, la poudre de cannelle utilisé comme charge particulaire libère au moins les COVs (E)-cinnamaldéhyde et $\gamma$-muurolene, a-muurolene. Dans un autre mode de réalisation préféré, la poudre de cannelle utilisée comme charge particulaire libère au moins les COVs (E)-cinnamaldéhyde et a-copaene. La présence de ces COVs peut aisément être vérifiée par chromatographie, notamment par chromatographie en phase gazeuse. Dans un mode de réalisation particulier, la poudre de cannelle utilisée présente un profil chromatographique similaire ou identique à l'un des profils de la figure 2B.

Dans un mode de réalisation particulier, la poudre de cannelle utilisée libère au moins les COVs (E)-cinnamaldéhyde et a-copaene, avec une aire de pic obtenue selon la méthode décrite dans l'exemple 1 supérieure à $100\times10^6$ pour (E)-cinnamaldéhyde et/ou supérieure à $30\times10^6$ pour l'$\alpha$,-copaene, préférentiellement une aire de pic supérieure à $200\times10^6$ pour (E)-cinnamaldéhyde et/ou une aire de pic supérieure à $60\times10^6$ pour l'a-copaene, encore plus préférentiellement une aire de pic supérieure à $350\times10^6$ pour (E)-cinnamaldéhyde et/ou une aire de pic supérieure à $90\times10^6$ pour l'a-copaene.

De manière comparable, l'invention a également pour objet un pansement comprenant une charge particulaire à base de curcuma, et notamment de *Curcuma longa,* de la famille des Zingibéracées. Le curcuma est une épice déjà utilisée dans le domaine médical, et notamment en applications topique, pour son action anti-inflammatoire. Les inventeurs ont mis en évidence que le curcuma en poudre est également apte à adsorber les odeurs lorsqu'il est utilisé dans un dispositif comprenant une couche absorbante destinée à être au contact de la source d'où émanent les mauvaises odeurs et une couche adsorbante dont la charge particulaire, qui n'est pas en contact direct avec la source de mauvaises odeurs, comprend du curcuma en poudre.

Dans un mode de réalisation, le curcuma est du *curcuma longa.* Un tel curcuma est disponible dans le commerce. Dans un mode de réalisation particulier, la charge particulaire dans le dispositif selon l'invention comprend ou consiste en de la poudre de curcuma libérant au moins le COV curcumène ou le COV tumérone. Préférentiellement, la poudre de curcuma utilisée libère au moins les COVs curcumène et tumérone. La présence de ces COVs peut aisément être vérifiée par chromatographie, notamment par chromatographie en phase gazeuse. Dans un mode de réalisation particulier, la poudre de curcuma utilisée présente un profil chromatographique similaire ou identique au profil de la figure 2C.

De manière comparable, l'invention a également pour objet un pansement comprenant une charge particulaire à base de clou de girofle. Dans un mode de réalisation particulier, la charge particulaire dans le dispositif selon l'invention comprend ou consiste en de la poudre de clou de girofle libérant au moins le COV eugénol. La présence de ce COV peut aisément être vérifiée par chromatographie, notamment par chromatographie en phase gazeuse. Dans un mode de réalisation particulier, la poudre de clou de girofle utilisée présente un profil chromatographique similaire ou identique au profil de la figure 2D.

L'invention a également trait à l'utilisation de cannelle, curcuma ou clou de girofle, tels que décrits ci-dessus, plus particulièrement sous forme de poudre, en tant qu'agent adsorbant dans un dispositif pour l'adsorption d'odeurs, et notamment d'odeurs corporelles. De même, l'invention a trait à l'utilisation d'une couche adsorbante comprenant un matériau perméable aux composés organiques volatils (COVs) et une charge particulaire adsorbante comprenant au moins de la cannelle, curcuma ou clou de girofle, tels que décrits ci-dessus, pour l'adsorption d'odeurs, et notamment d'odeurs corporelles. De telles utilisations sont particulièrement intéressantes dans le domaine médical et paramédical, notamment dans un dispositif médical tel qu'un pansement. De même, la poudre d'épice selon l'invention peut être utilisée dans des masques chirurgicaux ou des masques anti-odeurs. De tels masques peuvent être directement conçus à partir d'une couche adsorbante selon l'invention, intégrant une poudre d'épice telle que la cannelle, le curcuma ou le clou de girofle.

## Exemples

Exemple 1 : Comparaison des propriétés adsorbantes du charbon actif (Actisorb®), de la cannelle, du curcuma et du clou de girofle (en poudre).

Afin d'évaluer les propriétés adsorbantes de la cannelle (cannelle de Ceylan - FV), du curcuma (*curcuma longa*) et du clou de girofle, les profils d'adsorption sur trois COVs caractéristiques des nécroses malodorantes, à savoir le diméthyldisulfure (DMDS - $C_2H6S_2$), le phénol (Ph - $C_6H_6O$) et l'indole (In - $C_8H_7N$), ont été étudiés, en comparaison avec le profil d'adsorption du charbon actif qui est classiquement utilisé pour masquer les odeurs émanant de telles nécroses.

Parallèlement, les propriétés adsorbantes de trois cannelles de différentes provenances (FV), (FC) et (FI) ont été comparées en étudiant les profils d'adsorption sur ces trois mêmes COVs, en comparaison avec le profil d'adsorption du charbon actif.

## Matériel & Méthode

La composition de la solution test T3C, avec les trois COVs caractéristiques des nécroses malodorantes, est la suivante : eau pure contenant du diméthyldisulfure à 50mg/L (DMDS), du phénol à 125mg/L (Ph) et de l'indole à 500mg/L (In). Les composés purs pour la préparation de la solution proviennent de chez Sigma-Aldrich (Saint-Quentin Fallavier, France).

Les flacons échantillons (rond en verre, diamètre interne 22mm et hauteur 75mm, fermé de façon hermétique à l'aide d'une capsule à visser avec joint silicone) sont préparés de la façon suivante : dans le fond du flacon une matrice cellulose (carré 14mmx14mm, 100mg) sur laquelle est déposé un volume de 20µL de la solution T3C (soit 1µg DMDS, 2,5µg Ph et 10µg In) qui imbibe alors la matrice cellulose, puis recouvert d'un film d'Aquacel® en polyuréthane (diamètre 1,8cm, 40mg) et enfin mise en place d'un carré de cellulose (16mm×16mm, 130mg) contenant entre ses trames 30mg de charbon actif (Actisorb®) ou 30mg de cannelle en poudre de granulométrie moyenne 75µm, (*1(FV)/2(FC)/3(FI)*) ou 30mg de curcuma en poudre de granulométrie moyenne 90µm *(Curcuma longa)* ou 30mg de clou de girofle en poudre de granulométrie moyenne 820µm *(Fyzygium aromaticum,* ou giroflier). Un flacon témoin, la référence, est également préparé de la même façon mais ne contenant ni charbon actif ni épice.

La méthode d'analyse mise en œuvre couple une microextraction sur phase solide (SPME) en mode espace de tête (HS) gérée par un automate (bras passeur d'échantillons), à une chromatographie en phase gazeuse (GC) avec détection par spectrométrie de masse simple quadripole (MS).

La fibre SPME utilisée est une fibre carboxen- polydimethylsiloxane (CAR-PDMS), longueur 10mm et épaisseur 75µm, de chez Supelco (Sigma-Aldrich, Saint-Quentin Fallavier, France).

Le chromatographe et le spectromètre de masse sont respectivement les modèles GC 7890A et 5975C XL MSD de chez Agilent Technologies (Les Ulis, France).

L'automate passeur d'échantillons couplé au chromatographe est un MPS autosampler Gerstel (RIC, Saint-Priest, France).

Conditions opératoires : le flacon est incubé 6 min à 40°C puis l'extraction dans l'espace de tête du flacon est réalisée pendant 30 min toujours à 40°C. La désorption se fait dans l'injecteur du GC à 280°C en mode splitless durant 10 min. La séparation est réalisée sur une colonne DB5-MS, contenant un film de polydiméthylsiloxane (méthyl 95%-phényl 5%), de dimension 30mx0,25mm avec une épaisseur de film de 1μm, Le gaz vecteur est de l'Hélium avec un débit en sortie de colonne constant à 1,0mL/min. Le programme en température de la colonne est 40°C avec maintien 2 min, puis à 7°C/min jusqu'à 300°C et maintien 1 min (durée d'analyse 40,14 min).

En ce qui concerne la détection spectrométrique, les températures sont : pour la ligne de transfert 280°C, la source 230°C et le quad 150°C. L'ionisation se fait en impact électronique (EI) sous 70eV. Deux modes de détection mis en œuvre, le mode SCAN (balayage progressif) entre les m/z 25 à 300 et le mode SIM (Selected Ion Monitoring) pour les m/z 94 (ciblés DMDS et Ph) et 117 (ciblé In).

L'identification des composés est réalisée par comparaison des spectres de masse expérimentaux (pris sous le pic d'élution chromatographique) à ceux de la bibliothèque du National Institute of Standards and Technology (NIST, version 2.0f, rev.2010).

Les pics d'élution des composés sur le chromatogramme sont caractérisés : par leur temps de rétention donné en minute et pris au maximum du pic, ainsi que par leur aire (surface sous l'enveloppe du pic) donnée en unité d'aire arbitraire (représente un nombre de coups).

*Résultats*

Comme cela est représenté à la figure 1 et résumé dans le tableau 1 ci-dessous, le taux d'adsorption des COVs par le charbon actif (Actisorb®) est supérieur à 92%. La cannelle présente quant à elle un taux d'adsorption compris entre 80 et 92% en fonction des COVs, confirmant les propriétés adsorbantes des mauvaises odeurs de cette épice. De même les taux d'adsorption sont compris respectivement entre 67 et 90% pour le clou de girofle et entre 41 et 62% pour le curcuma.

Dans les tableaux 1 et 2, les aires de pic sont données en unité d'aire. Le tube témoin représente la référence en termes de quantités émises en COVs considérés dans le tube. Le taux d'adsorption est calculé selon : taux d'adsorption = [(aire du pic COV tube témoin) - (aire du pic COV tube avec épice)] / (aire du pic COV tube témoin).

Tableau 1 : Taux d'adsorption de diméthyldisulfure (DMDS), phénol (Ph) et indole (In) par 30 mg de charbon actif (Actisorb®) ou de cannelle (Cannelle de Ceylan - FV) ou de curcuma ou de clou de girofle (en poudre).

| COV (fragment m/z pour détection SIM) | Temps de rétention du COV (min) | Témoin Aire ($\times 10^6$) en Unité d'Aire (UA) | Actisorb® Aire ($\times 10^6$) % adsorbé | Cannelle FV Aire ($\times 10^6$) % adsorbé | Curcuma Aire (x10$^6$) % adsorbé | Clou de girofle Aire ($\times 10^6$) % adsorbé |
|---|---|---|---|---|---|---|
| DMDS (m/z = 94) | 10,05 | 118,7 | 0,5 99,6 % | 9,1 92,3 % | 44,7 62.3 % | 11,4 90,4 % |
| Ph (m/z = 94) | 16,14 | 88,2 | 5,7 93.5 % | 17,6 80,0 % | 47,7 45,9% | 27,9 68,4 % |
| In (m/z = 117) | 24,03 | 83,1 | 6,3 92,4 % | 10,5 87,4 % | 48,3 41,9 % | 27,4 67,0% |
| Témoin : 3COVs ; Actisorb® : 3COVs + 30mg charbon actif ; cannelle : 3COVs + 30mg poudre de cannelle ; Curcuma : 3COVs + 30mg poudre de curcuma ; clou de girofle : 3COVs + 30mg poudre de clou de girofle | | | | | | |

Tableau 2 : Taux d'adsorption de diméthyldisulfure (DMDS), phénol (Ph) et indole (In) par 30 mg de charbon actif (Actisorb®) ou de différentes cannelles en poudre (FV, FC et FI).

| Temps de rétention du COV (min) | COV (fragment m/z pour détection SIM) | Témoin Aire ($\times 10^6$) en UA | Actisorb Aire ($\times 10^6$) % **adsorbé** | Cannelle de Ceylan (FV) Aire ($\times 10^6$) % **adsorbé** | Cannelle FC Aire ($\times 10^6$) % **adsorbé** | Cannelle FI Aire ($\times 10^6$) % **adsorbé** |
|---|---|---|---|---|---|---|
| 10,00 | DMDS (m/z = 94) | 19.92 | 0.10 99 % | 5.99 70 % | 6.26 69 | 3.97 80 % |
| 16,10 | Ph (m/z = 94) | 12.34 | 0.09 99 % | 2.89 77 % | 3.07 75% | 3.32 73 % |
| 23,97 | In (m/z = 117) | 14.05 | 0.11 % | 1.52 89 % | 3.57 75 % | 2.27 84% |

Exemple 2 : Etude des COVs présents dans la cannelle, le curcuma et le clou de girofle

Comme cela a été mis en évidence ci-dessus, les poudres d'épice présentent un pouvoir adsorbant légèrement inférieur à celui du charbon actif Actisorb® vis-à-vis du diméthyldisulfure, du phénol et de l'indole. Cependant, sur un panel d'utilisateurs, ce sont les dispositifs selon l'invention, comprenant une charge particulaire à base de cannelle, qui remportent un plus fort taux de satisfaction (voir l'exemple 3 ci-dessous). Les épices odorantes présent en effet des COVs aptes à masquer au moins partiellement d'autres COVs, et notamment des COVs susceptibles d'émaner de plaies nauséabondes.

La combinaison, selon l'invention, des propriétés adsorbantes et odorantes des poudres d'épices utilisées permettent de potentialiser les effets du dispositif et de donner entière satisfaction pour supprimer les mauvaises odeurs.

Afin de déterminer quels sont les COVs présents dans la cannelle, le curcuma et le clou de girofle (en poudre), susceptibles de masquer les mauvaises odeurs, une analyse des COVs de la cannelle (cannelle de Ceylan), du curcuma (*curcuma longa*), du clou de girofle (*Fyzygium aromaticum*, ou giroflier) et du charbon actif (Actisorb®) a été réalisée. Pour cela, des flacons ont été préparés comprenant chacun directement 30 mg de cannelle (FV et FI), 30 mg de curcuma, 30mg de poudre de clou de girofle ou 30 mg d'Actisorb®. Puis les flacons ont été caractérisés avec la même méthode analytique que celle présentée dans l'exemple 1 (conditions opératoires rigoureusement identiques).

### Résultats

Comme cela est visible sur la figure 2, et résumé dans le tableau 3 ci-dessous, le charbon actif ne libère aucun COV (figure 2A). A l'inverse, les cannelles (figure 2B), comme le curcuma (figure 2C) et le clou de girofle (figure 2D) libèrent de nombreux COVs.

L'aspect masquant des épices odorantes, qui permet aux épices non seulement d'adsorber les mauvaises odeurs mais également de les couvrir, peut ainsi s'expliquer par la présence de COVs, totalement absents de l'Actisorb®, dont certains sont susceptibles de jouer fortement sur la perception sensorielle. C'est le cas par exemple du (E)-cinnamaldehyde pour la cannelle, du curcumène et du tumérone pour le curcuma ou encore de l'eugénol pour le clou de girofle.

Tableau 3 : Analyse comparative des COVs présents dans la cannelle (Cannelle de Ceylan - FV), le curcuma *(Curcuma longa),* le clou de girofle et le charbon actif (Actisorb®)

| Temps rétention (min) | Nom (numéro CAS) | Formule brute (masse molaire-g/mol) | Aire du pic si présent (en Unité d'Aire $\times 10^6$) | | | |
|---|---|---|---|---|---|---|
| | | | Actisorb® | Cannelle FV | Curcuma | Clou de Girofle |
| 17,65 | p-Cymene (99-87-6) | $C_{10}H_{14}$ (134) | *no* | *no* | 41 | *no* |
| 17,78 | D-Limonene (5989-27-5) | $C_{10}H_{16}$ (136) | *no* | 49 | 67 | *no* |
| 17,98 | Eucalyptol (470-82-6) | $C_{10}H_{18}O$ (154) | *no* | no | 30 | *no* |
| 20,93 | Camphor (76-22-2) | $C_{10}H_{16}O$ (152) | *no* | 37 | 61 | *no* |
| 21,78 | Methyl salicylate (ou Analgit) (119-36-8) | $C_8H_8O_3$ (152) | *no* | *no* | *no* | 128 |
| 23,66 | (E)-Cinnamaldehyde (14371-10-9) | $C_9H_8O$ (132) | *no* | 1660 | *no* | *no* |
| 25,40 | Eugenol (97-53-0) (164) | $C_{10}H_{12}O_2$ | *no* | *no* | *no* | 9059 |
| 25,76 | α-Copaene (3856-25-5) | $C_{15}H_{24}$ (204) | *no* | 3384 | *no* | 323 |
| 26,14 | (E)-Isoeugenol (5932-68-3) | $C_{10}H_{12}O_2$ (164) | *no* | *no* | *no* | 98 |
| 26,29 | (+)-Sativene (3650-28-0) | $C_{15}H_{24}$ (204) | *no* | 44 | *no* | *no* |
| 26,60 | α-Bergamotene (17699-05-7) | $C_{15}H_{24}$ (204) | *no* | 87 120 | *no* | *no* 4370 |
| 26,83 | Caryophyllene (87-44-5) | $C_{15}H_{24}$ (204) | *no* | | *no* | |
| 26,85 | D-germacrene (23986-74-5) | $C_{15}H_{24}$ (204) | *no* | 46 | *no* | *no* |
| 27,40 | α-Humulene (ou α-Caryophyllene) (6753-98-6) | $C_{15}H_{24}$ (204) | *no* | 205 | *no* | 833 |
| 27,43 | α-Curcumene (644-30-4) (202) | $C_{15}H_{22}$ | *no* | *no* | 862 | *no* |

(suite)

| Temps rétention (min) | Nom (numéro CAS) | Formule brute (masse molaire-g/mol) | Aire du pic si présent (en Unité d'Aire $\times 10^6$) | | | |
|---|---|---|---|---|---|---|
| | | | Actisorb® | Cannelle FV | Curcuma | Clou de Girofle |
| 27,57 | $\gamma$-Muurolene (30021-74-0) | $C_{15}H_{24}$ (204) | no | 437 | no | 108 |
| 27,63 | $\alpha$-Zingiberene (495-60-3) | $C_{15}H_{24}$ (204) | no | no | 162 | no |
| 27,86 | Aceteugenol (93-28-7) | $C_{12}H_{14}O_3$ (206) | no | no | no | 149 |
| 27,89 | $\alpha$-Himachalene (3853-83-6) | $C_{15}H_{24}$ (204) | no | no | 124 | no |
| 27,96 | $\alpha$-Muurolene (31983-22-9) | $C_{15}H_{24}$ (204) | no | 544 | no | no |
| 28,05 | $\beta$-selinene (17066-67-0) | $C_{15}H_{24}$ (204) | no | 41 | no | no |
| 28,12 | $\alpha$-selinene (473-13-2) | $C_{15}H_{24}$ (204) | no | 74 | no | no |
| 28,24 | $\beta$-Sesquiphellandrene (20307-83-9) | $C_{15}H_{24}$ (204) | no | no | 277 | no |
| 28,28 | $\delta$-Cadinene (483-76-1) | $C_{15}H_{24}$ (204) | no | 908 | no | 179 |
| 28,45 | (-)-Calamene (483-77-2) | $C_{15}H_{22}$ (202) | no | 646 | no | no |
| 28,62 | Isolongifolene, 4,5,9,10-dehydro (156747-45-4) | $C_{15}H_{20}$ $C_{15}H_{20}$ (200) | no | 167 | no | no |
| 28,86 | $\alpha$-Calacorene (21391-99-1) | $C_{15}H_{20}$ (200) | no | 132 | no | no |
| 29,64 | Tumerone (180315-67-7) | $C_{15}H_{22}O$ (218) | no | no | 82 | no |
| 30,38 | Cubenol (21284-22-0) | $C_{15}H_{26}O$ (222) | no | 45 | no | no |
| 30,63 | $\alpha$-Cadinol (481-34-5) | $C_{15}H_{26}O$ (222) | no | 48 | no | no |
| 30,70 | $\beta$-Tumerone (82508-14-3) | $C_{15}H_{22}O$ (218) | no | no | 841 | no |
| 30,78 | AR-Tumerone (532-65-0) | $C_{15}H_{20}O$ (216) | no | no | 167 | no |
| 31,13 | Cadalene (483-78-3) | $C_{15}H_{18}$ (198) | no | 37 | no | no |
| 31,37 | $\alpha$-Tumerone (82508-15-4) | $C_{15}H_{22}O$ (218) | no | no | 225 | no |
| no = non observé | | | | | | |

Des cannelles de différentes origines (FV, FC, FI, W) ont également été analysées, afin d'identifier les COVs caractéristiques.

Tableau 4 : Analyse comparative des COVs présents dans différentes cannelles

| Tr (min) | Nom (numéro CAS) | Formule brute | MW (g/mol) | Aire (x$10^6$) en Unité d'Aire | | | |
|---|---|---|---|---|---|---|---|
| | | | | Cannelle FV (cannelle de Ceylan) | Cannelle FC | Cannelle FI | Cannelle W |
| 19,30 | L-Linalool (78-70-6) | $C_{10}H_{18}O$ | 154 | nd | trace | 30 | 118 |
| 23,63 | (E)-Cinnamaldehyde (14371-10-9) | $C_9H_8O$ | 132 | 88 | 658 | 656 | 367 |
| 25,83 | $\alpha$-Copaene (3856-25-5) | $C_{15}H_{24}$ | 204 | 1431 | 62 | 726 | 117 |

(suite)

| Tr (min) | Nom (numéro CAS) | Formule brute | MW (g/mol) | Aire (x10^6) en Unité d'Aire | | | |
|---|---|---|---|---|---|---|---|
| | | | | Cannelle FV (cannelle de Ceylan) | Cannelle FC | Cannelle FI | Cannelle W |
| 26,81 | β-Caryophyllene (87-44-5) | $C_{15}H_{24}$ | 204 | 8 | 3 | 307 | 948 |
| 27,63 | γ-Muurolene (30021-74-0) | $C_{15}H_{24}$ | 204 | 79 | 9 | 32 | nd |
| 28,03 | α-Muurolene (31983-22-9) | $C_{15}H_{24}$ | 204 | 103 | 28 | 79 | 2 |
| 28,37 | δ-Cadinene (483-76-1) | $C_{15}H_{24}$ | 204 | 165 | 35 | 86 | 5 |
| 28,51 | (-)-Calamene (483-77-2) | $C_{15}H_{22}$ | 202 | 142 | 27 | 61 | 6 |
| nd = non détecté | | | | | | | |

On observe ainsi que ces différentes cannelles présentent une aire de pic pour le (E)-Cinnamaldehyde supérieure à $350 \times 10^6$ et/ou une aire de pic pour le α-Copaene supérieure à $100 \times 10^6$.

Exemple 3 : Perception sensorielle

Les propriétés adsorbantes et masquantes de la cannelle (cannelle de Ceylan - FV) et du charbon actif ont été évaluées et comparées par tests à l'aveugle sur un panel d'utilisateurs sur des odeurs émises par du fromage Maroille. Parallèlement, les propriétés adsorbantes et masquantes de différentes cannelles (FV, FC, FI et W) ont été comparées, également par tests à l'aveugle sur ce panel d'utilisateurs sur des odeurs émises par du fromage Maroille.

En effet, selon l'analyse de JP Dumont (JP Dumont et al., Le Lait/ Janvier-Février 1974/ N°531-532, « Etude des composés volatils neutres présents dans les fromages à pâte molle et à croute lavée »), l'odeur du Maroille se rapproche sensiblement de celle des plaies tumorales. Le tableau 3 ci-dessous récapitule les principaux COVs libérés par les plaies tumorales et par le Maroille. Les COVs libérés par les plaies tumorales ont été obtenus par analyse par chromatographie en phase gazeuse (méthode d'analyse identique à celle de l'exemple 1) de trois échantillons de nécrose (nécroses prélevées sur des plaies tumorales malodorantes).

Tableau 5 : Principaux COVs communs au Maroille et aux plaies tumorales

| Composés | Maroilles | Plaies tumorales | Perception sensorielle |
|---|---|---|---|
| 3-méthyl-1-butanol/ Acide butanoïque | ++ | +++ | Odeur forte de fromage de chèvre |
| Diméthyle disulfure | ++ | +++ | Odeur d'ail pourri |
| Acétate d'éthyle / Acide acétique | +++ | +++ | Odeur de vinaigre |
| Acétophénone / phénol | ++++ | ++ | Odeur de goudron |
| Indole | ++++ | ++++ | Odeur intense de matière fécale |

**Matériel & Méthode**

L'enquête a été réalisée au sein de l'Institut Curie. Les 16 participants de l'enquête sont des soignants, des médecins et des chercheurs de l'institut Curie.

L'objectif principal était de déterminer la ou les meilleures conditions permettant de diminuer la perception des odeurs malodorantes au moyen de poudre de cannelle.

L'évaluation du caractère hédonique des échantillons s'est fait sur une échelle de 1 (très désagréable) à 5 (très agréable), le 3 étant neutre. Les odeurs perçues ont également été qualifiées par les adjectifs : «lourde / forte / collante / aromatique / écoeurante / dérangeante / agréable / sucrée », et associées aux adjectifs comme « cadavérique / putride / urineuse / excrémentielle / végétale / pestilentielle » (réponse multiple possible).

Echantillons

Les échantillons comprenaient chacun 6 g de Maroille pour 1g d'épice ou d'Actisorb®. Le Contrôle, ou témoin, consistait en 6g de Maroille, seul.

Ces échantillons ont été placés dans des pots en verre entourés de papier aluminium pendant au moins 20-30 min avant le test, afin que les odeurs des échantillons se dispersent dans l'air des bocaux et permettent une évaluation sensorielle efficace.

Déroulement de l'étude :

Les participants ont été prévenus du caractère désagréable des échantillons afin d'éviter un effet de surprise ou de retenu, et maintenir ainsi une inhalation constante.

Les tests ont été faits en aveugle. Les participants ne connaissaient pas le contenu des échantillons. Les échantillons ont été présentés à chacun des participants.

Un « rinçage de nez » a été systématiquement réalisé entre deux évaluations. Le rinçage de nez s'effectuait en reniflant un bocal contenant de l'eau ou sa propre peau (poignet ou coude).

L'enquête de perception s'est déroulée en cinétique pour évaluer l'efficacité des composés sur les COVs malodorants. Les temps de cinétique étaient T0, T24 et T48 (en heures).

L'analyse statistique a été réalisée avec l'aide du Pr C. Dacremont, Centre des Sciences du Goût et de l'Alimentation, CNRS - INRA- Université de Bourgogne - AgroSup Dijon.

*Résultats*

La majorité des participants ont considéré les odeurs émanant des échantillons contrôles (Maroille seul) et des échantillons Actisorb® (Maroille + Actisorb®) comme désagréables (2) à très désagréables (1). Plus précisément, les participants associent à 68% l'échantillon témoin comme très désagréable et à 53% l'échantillon contenant l'Actisorb® comme désagréable (figures 3A et 3B).

A l'inverse, la majorité des participants ont considéré les odeurs émanant des échantillons contenant les épices comme neutres (3), agréables (4) ou très agréables (5). Notamment, 40% des participant ont associé l'échantillon Cannelle comme agréable, et 18% pour le curcuma. (figures 3C, 3D et 3E).

Les odeurs émanant des échantillons contenant les épices ont été principalement qualifiées comme étant aromatiques et agréables. A l'inverse, les odeurs émanant des échantillons témoins comme les échantillons contenant l'Actisorb® ou le contrôle ont été qualifiées d'écœurantes, dérangeantes et fortes (Figure 4). De même, si un grand nombre de participants a associé l'odeur émanant des échantillons contenant une épice à une odeur « végétale » ou « gourmande », les qualificatifs utilisés pour les odeurs émanant de l'échantillon contrôle et de l'échantillon contenant l'Actisorb® sont principalement « pourrie » et « pestilentielle » (Figure 5).

Les tests sensoriels réalisés avec les différentes cannelles ont tous donné satisfaction (figure 6). La majorité des participants ont considéré les odeurs émanant des échantillons contenant les cannelles comme neutres (3), agréables (4) ou très agréables (5).

Ces tests sensoriels confirment que les épices utilisées ont un fort pouvoir adsorbant et masquant des COVs et notamment des COVs perçus comme désagréables par les utilisateurs. A l'inverse, le charbon actif n'est pas à même d'adsorber et masquer de manière satisfaisante ces mêmes COVs, qui continuent d'être perçus comme désagréables et/ou gênants par les utilisateurs.

**Revendications**

**1.**  Dispositif pour l'adsorption d'odeurs comprenant une couche absorbante et une couche adsorbante, ladite couche adsorbante étant munie d'un matériau perméable aux composés organiques volatils (COVs) et d'une charge particulaire adsorbante comprenant au moins une épice, choisie parmi la cannelle, le curcuma et le clou de girofle, ledit matériau perméable aux COVs étant un textile tissé ou non tissé, ledit dispositif comprenant entre 0,5 et 5 g d'épices pour 100 cm$^2$ de matériau perméable aux COVs, ladite épice étant maintenue sous forme de poudre dans ledit dispositif.

**2.**  Dispositif pour l'adsorption d'odeurs selon la revendication 1, dans lequel la charge particulaire adsorbante consiste en au moins une épice choisie parmi la cannelle, le curcuma et le clou de girofle, préférentiellement la cannelle.

3. Dispositif pour l'adsorption d'odeurs selon la revendication 1 ou 2, ledit dispositif étant apte à adsorber des odeurs corporelles.

4. Dispositif pour l'adsorption d'odeurs selon l'une des revendications précédentes, dans lequel la charge particulaire présente une granulométrie moyenne inférieure à 1000µm, 900µm, 800µm, 700µm, 600µm, 500µm, 400µm, 300µm, 200µm, 150µm, 100µm, 90µm, 80µm, 70 µm, 60 µm, 50 µm, 40 µm.

5. Dispositif pour l'adsorption d'odeurs selon l'une des revendications 1 à 3, dans lequel la charge particulaire présente une granulométrie moyenne comprise entre 700 et 900 µm.

6. Dispositif pour l'adsorption d'odeurs selon l'une des revendications 1 à 3, dans lequel la charge particulaire présente une granulométrie moyenne comprise entre 40 et 100 µm.

7. Dispositif pour l'adsorption d'odeurs selon l'une des revendications précédentes, comprenant entre 1 et 4 g, préférentiellement 2g d'épices pour 100 cm$^2$ de matériau perméable aux COVs.

8. Dispositif pour l'adsorption d'odeurs selon l'une des revendications précédentes, dans lequel le matériau perméable aux COVs est du nylon, du coton, du lin, de la viscose, du polyéthylène, du polypropylène ou du polyester.

9. Dispositif pour l'adsorption d'odeurs selon l'une quelconque des revendications précédentes, dans lequel le matériau perméable aux COVs présente des formes géométriques, et notamment un quadrillage ou des alvéoles, dans lesquelles la charge particulaire adsorbante est apte à être contenue.

10. Dispositif pour l'adsorption d'odeurs selon l'une quelconque des revendications précédentes, dans lequel la couche absorbante comprend au moins un matériau perméable ou semi-perméable, préférentiellement choisi parmi les alginates, le carboxyméthylcellulose (CMC), le coton hydrophile et les polymères absorbants, tels que la mousse de polyuréthane.

11. Dispositif pour l'adsorption d'odeurs selon l'une quelconque des revendications précédentes, comprenant en outre une couche protectrice, préférentiellement semi-perméable ou imperméable à l'humidité, prise en sandwich entre la couche absorbante et la couche adsorbante.

12. Dispositif pour l'adsorption d'odeurs selon l'une quelconque des revendications précédentes, comprenant deux couches absorbantes et dans lequel la couche adsorbante est prise en sandwich entre les deux couches absorbantes.

13. Dispositif pour l'adsorption d'odeurs selon l'une des revendications 1-12, sous forme de pansement.

14. Dispositif pour l'adsorption d'odeurs selon l'une des revendications 1-12, sous forme de couche, de protection hygiénique, compresse absorbante ou de protège-slip.

**Patentansprüche**

1. Vorrichtung zur Adsorption von Gerüchen, umfassend eine Absorptionsschicht und eine Adsorptionsschicht, wobei die Adsorptionsschicht mit einem für flüchtige organische Verbindungen (VOCs) permeablen Material und mit einem adsorbierenden partikelförmigen Füllstoff, umfassend wenigstens ein Gewürz, ausgewählt aus Zimt, Curcuma und Gewürznelke, versehen ist, wobei das für VOCs permeable Material ein gewebter oder nicht gewebter Stoff ist, wobei die Vorrichtung zwischen 0,5 und 5 g Gewürze pro 100 cm$^2$ VOCs permeables Material umfasst, wobei das Gewürz in Pulverform in der Vorrichtung vorliegt.

2. Vorrichtung zur Adsorption von Gerüchen gemäß Anspruch 1, wobei der adsorbierende partikelförmige Füllstoff aus wenigstens einem Gewürz, ausgewählt aus Zimt, Curcuma und Gewürznelke, vorzugsweise Zimt besteht.

3. Vorrichtung zur Adsorption von Gerüchen gemäß Anspruch 1 oder 2, wobei die Vorrichtung geeignet ist, Körpergerüche zu adsorbieren.

4. Vorrichtung zur Adsorption von Gerüchen gemäß einem der vorhergehenden Ansprüche, wobei der partikelförmige Füllstoff eine mittlere Partikelgröße kleiner 1000 µm, 900 µm, 800 µm, 700 µm, 600 µm, 500 µm, 400 µm, 300

μm, 200 μm, 150 μm, 100 μm, 90 μm, 80 μm, 70 μm, 60 μm, 50 μm, 40 |im aufweist.

5. Vorrichtung zur Adsorption von Gerüchen gemäß einem der Ansprüche 1 bis 3, wobei der partikelförmige Füllstoff eine mittlere Partikelgröße zwischen 700 und 900 μm umfasst.

6. Vorrichtung zur Adsorption von Gerüchen gemäß einem der Ansprüche 1 bis 3, wobei der partikelförmige Füllstoff eine mittlere Partikelgröße zwischen 40 und 100 μm umfasst.

7. Vorrichtung zur Adsorption von Gerüchen gemäß einem der vorhergehenden Ansprüche, umfassend zwischen 1 und 4 g, vorzugsweise 2 g Gewürze pro 100 cm$^2$ VOCs permeables Material.

8. Vorrichtung zur Adsorption von Gerüchen gemäß einem der vorhergehenden Ansprüche, wobei das VOCs permeable Material Nylon, Baumwolle, Leinen, Viskose, Polyethylen, Polypropylen oder Polyester ist.

9. Vorrichtung zur Adsorption von Gerüchen gemäß einem der vorhergehenden Ansprüche, wobei das VOCs permeable Material geometrische Formen und insbesondere ein Gitter oder Zellen aufweist, in denen der adsorbierende partikelförmige Füllstoff enthalten sein kann.

10. Vorrichtung zur Adsorption von Gerüchen gemäß einem der vorhergehenden Ansprüche, wobei die Absorptionsschicht wenigstens ein permeables oder semipermeables Material, vorzugsweise ausgewählt aus Alginaten, Carboxymethylcellulose (CMC), Watte und absorbierenden Polymeren, wie Polyurethanschaum, umfasst.

11. Vorrichtung zur Adsorption von Gerüchen gemäß einem der vorhergehenden Ansprüche, umfassend außerdem eine Schutzschicht, die vorzugsweise semipermeabel oder impermeabel für Feuchtigkeit ist und sandwichartig zwischen Absorptionsschicht und Adsorptionsschicht angeordnet ist.

12. Vorrichtung zur Adsorption von Gerüchen gemäß einem der vorhergehenden Ansprüche, umfassend zwei Absorptionsschichten und wobei die Adsorptionsschicht sandwichartig zwischen den beiden Absorptionsschichten angeordnet ist.

13. Vorrichtung zur Adsorption von Gerüchen gemäß einem der Ansprüche 1 bis 12 in Form eines Verbands.

14. Vorrichtung zur Adsorption von Gerüchen gemäß einem der Ansprüche 1 bis 12 in Form einer Windel, eines Hygieneschutzes, einer Saugeinlage oder Slipeinlage.

**Claims**

1. Device for adsorbing odours comprising an absorbent layer and an adsorbent layer, said adsorbent layer being provided with a material permeable to volatile organic compounds (VOCs) and an adsorbent particulate filler comprising at least one spice, chosen from among cinnamon, turmeric and clove, said material permeable to VOCs being a woven or non-woven textile, said device comprising between 0.5 and 5 g of spice for 100 cm$^2$ of material permeable to VOCs, said spice being held in the form of powder in said device.

2. Device for adsorbing odours according to claim 1, wherein the adsorbent particulate filler consists of at least one spice chosen from among cinnamon, turmeric and clove, preferably cinnamon.

3. Device for adsorbing odours according to claim 1 or 2, said device being capable of adsorbing body odours.

4. Device for adsorbing odours according to one of the preceding claims, wherein the particulate filler has an average particle size less than 1000 μm, 900 μm, 800 μm, 700 μm, 600 μm, 500 μm, 400 μm, 300 μm, 200 μm, 150 μm, 100 μm, 90 μm, 80 μm, 70 μm, 60 μm, 50 μm, 40 μm.

5. Device for adsorbing odours according to one of claims 1 to 3, wherein the particulate filler has an average particle size between 700 and 900 μm.

6. Device for adsorbing odours according to one of claims 1 to 3, wherein the particulate filler has an average particle size between 40 and 100 μm.

7. Device for adsorbing odours according to one of the preceding claims, comprising between 1 and 4 g, preferably 2 g of spices for 100 cm$^2$ of material permeable to VOCs.

8. Device for adsorbing odours according to one of the preceding claims, wherein the material permeable to VOCs is nylon, cotton, linen, viscose, polyethylene, polypropylene or polyester.

9. Device for adsorbing odours according to any one of the preceding claims, wherein the material permeable to VOCs has geometric shapes, and in particular a grid or alveolus, in which the adsorbent particulate filler is able to be contained.

10. Device for adsorbing odours according to any one of the preceding claims, wherein the absorbent layer comprises at least one permeable or semi-permeable material, preferably chosen from among alginates, carboxymethylcellulose (CMC), hydrophilic cotton and absorbent polymers, such as polyurethane foam.

11. Device for adsorbing odours according to any one of the preceding claims, further comprising a protective layer that is preferably semi-permeable or impermeable to moisture, sandwiched between the absorbent layer and the adsorbent layer.

12. Device for adsorbing odours according to any one of the preceding claims, comprising two absorbent layers and wherein the adsorbent layer is sandwiched between the two absorbent layers.

13. Device for adsorbing odours according to one of claims 1-12, in the form of a dressing.

14. Device for adsorbing odours according to one of claims 1-12, in the form of a nappy, sanitary protection, absorbent pad or pantiliner.

FIGURE 1

A

B

FIGURE 2

C

D

FIGURE 2 (suite)

FIGURE 3

**Contrôle**

**Charbon**

**Cannelle**

**Curcuma**

**Clou de girofle**

FIGURE 4

FIGURE 5

FIGURE 6

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1352927 A

**Littérature non-brevet citée dans la description**

- **PRETI et al.** *Journal of Chromatography B,* 2009, vol. 877, 2011-2018
- **REY et al.** *International Journal of Greenhouse Gas Control,* 2013, vol. 19 (11), 576-583
- **JP DUMONT et al.** *Etude des composés volatils neutres présents dans les fromages à pâte molle et à croute lavée,* Janvier 1974